# EUROPÄISCHE PATENTSCHRIFT

(11) **EP 0 552 758 B1**
(45) Veröffentlichungstag und Bekanntmachung des Hinweises auf die Patenterteilung: **11.06.1997**
(21) Anmeldenummer: 93100883.3
(22) Anmeldetag: 21.01.1993
(51) Int. Cl.: C07D 239/54, C07D 239/48

(54) **N-5-geschützte 2,5-Diamino-4,6-dichlorpyrimidine und Verfahren zu deren Herstellung**
N-5-protected 2,5-diamino-4,6-dichloropyrimidines and process for their preparation
2,5-Diamino-4,6-dichloropyrimidines N-5-protégées et procédé pour leur préparation

(30) Priorität: 22.01.1992 CH 179/92
(43) Veröffentlichungstag der Anmeldung: 28.07.1993
(73) Patentinhaber: LONZA AG, CH-3945 Gampel/Wallis (CH)
(72) Erfinder: Stucky, Gerhard, Dr., Brig-Glis (Kanton Wallis) (CH); Previdoli, Felix, Dr., Brig-Glis (Kanton Wallis) (CH)
(74) Vertreter: Weinhold, Peter, Dr.

(56) Entgegenhaltungen:
- WO-A-91/01310
- GB-A- 2 116 554
- SYNTHESIS Nr. 7, Juli 1990, STUTTGART Seiten 587 - 589 MICHEL LEGRAVEREND ET AL. 'A new Route to 2,5-Diamino-4,6-dichloropyrimidine, A key precursor of 9-Substituted Guanines'
- THEODORA W. GREENE 'Protective groups in Organic Synthesis' 1981 , JOHN WILEY & SONS , NEW YORK

## Beschreibung

Die Erfindung betrifft N-5-geschützte 2,5-Diamino-4,6-dichlorpyrimidine der allgemeinen Formel 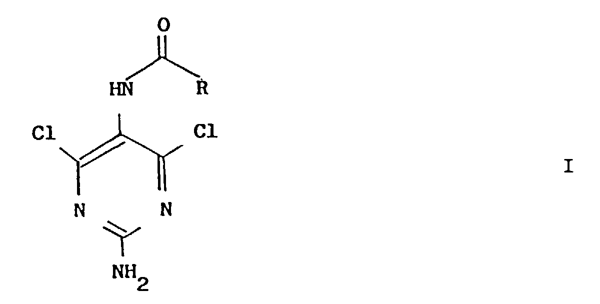 worin R eine C₁-C₄-Alkoxygruppe oder eine Trifluormethylgruppe bedeutet, sowie ein Verfahren zu deren Herstellung. Diese Verbindungen bilden wertvolle Zwischenprodukte zur Herstellung von antiviralen Nucleotidderivaten(vgl. WO-A-91/01 310).

Es sind verschiedene Synthesen zur Herstellung von 2,5-Diamino-4,6-dichlorpyrimidinen bekannt. So berichten Legraverend et al. in Synthesis 1990, S. 587, dass sich ein Aminomalonsäureethylester mit Guanidin in Gegenwart von Natriumalkoholat in einer Ausbeute von 64% zum 4,6-Dihydroxy-2,5-diaminopyrimidin ringschliessen lässt. Das Dihydroxypyrimidin wird dann mit Phosphoroxychlorid/Phosphorpentachlorid in Gegenwart eines quaternären Ammoniumsalzes bei drastischen Reaktionsbedingungen in einer Ausbeute von 32% zum gewünschten Dichlorpyrimidin umgesetzt.

Legraverend gibt an, dass die Chlorierung mit Phosphoroxychlorid nicht erfolgreich verläuft. Die Notwendigkeit von Zusatzreagentien wie Phosphorpentachlorid, von quaternären Ammoniumsalzen oder von drastischen Reaktionsbedingungen und die damit erreichbare bescheidene Ausbeute, ist aber ein offensichtlicher Nachteil dieser Synthese. Eine sprunghafte Verbesserung der Synthese wurde in der PCT-Anmeldung WO-A-91/01 310 offenbart. Der Chlorierungsschritt vom 4,6-Dihydroxy-2,5-diaminopyrimidin zum gewünschten 4,6-Dichlorderivat wird darin mit Phosphoroxychlorid alleine als Chlorierungsmittel in Gegenwart eines quaternären Ammoniumchlorids oder einem Hydrochlorid eines tert. Amins durchgeführt.
Die Ausbeuten konnten auf 50 - 60% gesteigert werden. Die Anwendung von beträchtlichen Mengen an Ammoniumsalzen oder Aminsalzen, die als Salzlast anfallen und einer Entsorgung zugeführt werden müssen, bildet aber nach wie vor einen beträchtlichen Nachteil.

Die Aufgabe bestand folglich darin, eine Synthese zu entwickeln, die die genannten Nachteile nicht aufweist.

Die Aufgabe konnte gelöst werden mit den N-5-geschützten 2,5-Diamino-4,6-dichlorpyrimidinderivaten nach Anspruch 1 sowie mit dem erfindungsgemässen Verfahren nach Anspruch 2.

Die erfindungsgemässen N-5-geschützten 2,5-Diamino-4,6-dichlorpyrimidine sind bisher nicht beschriebene Verbindungen. Bevorzugte Vertreter dieser Verbindungen der allgemeinen Formel I sind die N-5-(C₁-C₄)Alkoxycarbonyl-2,5-diamino-4,6-dichlorpyrimidine (R = C₁-C₄-alkoxy).

Erfindungsgemäss wird im ersten Verfahrensschritt ein N-geschützter Aminomalonsäureester der allgemeinen Formel 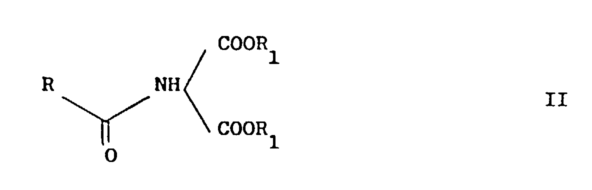 worin R₁ eine C₁-C₄-Niederalkylgruppe bedeutet und R die genannte Bedeutung hat, mit Guanidin in Gegenwart eines Alkalialkoholats zum N-5-geschützten-4,6-Dihydroxy-2,5-diaminopyrimidin der allgemeinen Formel 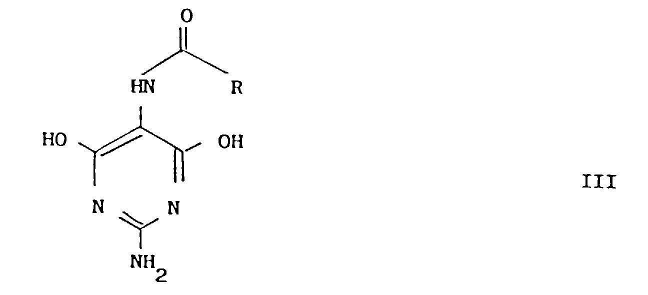 worin R die genannte Bedeutung hat, ringgeschlossen.
Diese Verbindungen sind bisher nicht beschrieben und deshalb ebenfalls Bestandteil der Erfindung.

Die als Ausgangsprodukte eingesetzten N-geschützten Aminomalonsäureester der allgemeinen Formel II können auf einfache Weise aus einem Aminomalonsäureester, im Fall wo R eine Alkoxygruppe bedeutet, durch Umsetzung mit einem entsprechenden Halogenameisensäurealkylester, oder im Fall, wo R eine Trifluormethylgruppe bedeutet, durch Umsetzung mit einem Trifluoracetylhalogenid hergestellt werden.

Das Alkalialkoholat wird zweckmässig in situ aus dem jeweiligen Alkalimetall und dem jeweiligen Alkohol hergestellt. Üblicherweise setzt man den Alkohol im Überschuss ein, wobei der überschüssige Alkohol gleich als Lösungsmittel dient.

Vorzugsweise wird die Umsetzung in Gegenwart von in situ gebildetem Natriummethylat in Methanol oder Natriumethylat in Ethanol durchgeführt.

Zweckmässig erfolgt die Ringschlussreaktion bei einer Temperatur zwischen Raumtemperatur und 80°C, vorzugsweise bei Rückflussbedingungen.

Die Isolation des N-5-geschützten 4,6-Dihydroxy-2,5-diaminopyrimidins der allgemeinen Formel III kann auf fachmännische Weise erfolgen.

In der Folge wird das N-5-geschützte 4,6-Dihydroxy-2,5-diaminopyrimidin erfindungsgemäss mit Phosphoroxychlorid zum gewünschten Endprodukt der allgemeinen Formel I chloriert.

Die Chlorierung mit Phosphoroxychlorid erfolgt überraschenderweise ohne Zusatzstoffe bei einer Temperatur von zweckmässig 80°C bis Rückflusstemperatur des Phosphoroxychlorids, vorzugsweise bei 80°C bis 90°C. Das Phosphoroxychlorid dient dabei im Überschuss eingesetzt, zusätzlich als Lösungsmittel. Ein zusätzliches inertes Lösungsmittel kann zwar zugesetzt werden, bringt aber keine Vorteile.
Die Chlorierung ist ist in der Regel nach maximal 6 h beendet.

Nach fachmännisch üblicher Aufarbeitung kann das N-5-geschützte 4,6-Dichlor-2,5-diaminopyrimidin in guter Ausbeute und Reinheit isoliert werden.

### Beispiel 1:

### a) N-Butyloxycarbonyl-aminomalonsäurediethylester (R = OBu)

Zu einer eiskalten Suspension von 15 g (70,87 mmol) Aminomalonsäureethylester-hydrochlorid in 180 ml Methylenchlorid wurden innerhalb einiger Minuten 10,8 g (75 mmol) Chlorameisensäurebutylester und anschliessend 14,7 g (145 mmol) Triethylamin zugegeben. Dabei wurde die Temperatur stets unter 20°C gehalten. Nach beendeter Zugabe wurde die Reaktion für weitere 75 min bei Raumtemperatur gerührt. Die Reaktionslösung wurde mit 80 ml CH₂Cl₂ verdünnt, mit 2 Portionen Wasser gewaschen und über Na₂SO₄ getrocknet. Nach Einengen am Rotationsverdampfer erhielt man 18,8 g (96%) reines Titelprodukt.
- ¹H-NMR (CDCl₃, 300 MHz) δ in ppm:: 0,92 (t, 3H);
1,31 (t, 6H);
1,3-1,45 (m, 2H);
1,55-1,65 (m, 2H);
4,1 (t, 2H);
4,2-4,4 (m, 4H);
5,0 (d, 1H);
5,1 (d, 1H);
5,7 (d, 1H);
5,95 (d, 1H).

### b) 5-(N-Butyloxycarbonyl)-2-amino-4,6-dihydroxypyrimidin (R = OBu)

Zu einer Lösung von 4,2 g (180 mmol) Natrium in 130 ml Ethanol wurden 11 g (115 mmol) Guanidinhydrochlorid zugegeben. Nach 10 min wurde die Suspension mit 18 g (65 mmol des Produktes aus Stufe a)) versetzt und zum Rückfluss erhitzt. Nach einer Stunde wurde die Reaktionslösung auf 40°C abgekühlt, mit konz. HCl auf pH 2 gestellt, und das Ethanol am Rotationsverdampfer grösstenteils abdestilliert. Der Eindampfrückstand wurde in 100 ml H₂O aufgeschlämmt, im Eisbad gekühlt und anschliessend das Produkt abfiltriert. Nach Waschen mit 3 Portionen kaltem Wasser und Trocknen im Vakuumtrockenschrank bei 100 °C erhielt man 13,45 g (85%) Titelprodukt.
- ¹H-NMR (DMSO, 300 MHz) δ in ppm:: 0,9 (t, 3H);
1,1-1,6 (m, 4H);
3,85-3,95 (m, 2H);
6,22 (2s, 2H);
7,0 (2s, 2H);
7,08 (2s, 1H);
7,3 (2s, 1H);
10,0-11,0 (br, 2H).

### c) 5-(N-Butyloxycarbonyl)-2-amino-4,6-dichlorpyrimidin R = OBu)

Eine Suspension von 3 g (12,39 mmol) des Produktes aus Stufe b) in 25 ml Phosphoroxychlorid wurde 2 Stunden auf 90°C erhitzt. Dabei ging der Feststoff langsam in Lösung. Das überschüssige POCl₃ wurde am Rotationsverdampfer eingeengt, und der Rückstand auf Eis gegossen. Dabei stieg die Temperatur auf 50°C an. Bei dieser Temperatur wurde auf pH 3 gestellt und eine weitere Stunde gerührt. Die Suspension wurde auf 5°C abgekühlt und das Produkt filtriert. Nach Trocknen im Vakuum-trockenschrank bei 40°C erhielt man 2 g (57%) Titelprodukt mit einem Gehalt von 96% (nach GC/Fl%).
Schmp.: 161-164°C
¹H-NMR (CDCl₃, 300 MHz) δ in ppm: 0,8-1,1 (m 3H);
1,2-1,9 (m, 4H);
4,1-4,3 (m, 2H);
5,4 (s, 2H);
5,9-6,2 (m, 1H).

| | | | |
|---|---|---|---|
| Anal.ber. für C₉H₁₂Cl₂N₂O₂ (279): | C = 37,99 | H = 4,3 | N = 20,0 |
| gef.: | C = 37,7 | H = 4,4 | N = 19,6 |

### Beispiel 2:

### a) 5-(N-Methoxycarbonyl)-2-amino-4,6-dihydroxypyrimidin (R = OMe)

Zu einer Suspension von 30 g (0,128 mol) N-Methoxycarbonylaminomalonsäurediethylester und 22 g (0,23 mol) Guanidinhydrochlorid in 300 ml Methanol wurden 60 g einer 30% Natriummethanolatlösung in Methanol innerhalb von 5 min zugetropft. Anschliessend wurde die Suspension während 160 min am Rückfluss erhitzt. Das Methanol wurde am Rotationsverdampfer eingeengt, der Rückstand in 150 ml H₂O aufgenommen, der pH auf 3 - 4 eingestellt, und nach Abkühlen auf 5°C wurde das Produkt abfiltriert, drei Mal mit je 30 ml H₂O gewaschen und am Hochvakuum bei 100°C getrocknet. Man erhielt 17,3 g (67%) des Titelproduktes.
- ¹H-NMR: (DMSO, 300 MHz) δ in ppm:: 3,55 (s, 3H);
6,7 (s, 2H);
7,2 (s, 1H);
7,45 s, 1H);
10,0-11,0 (br, 2H).

### b) 5-(N-Methoxycarbonyl)-2-amino-4,6-dichlorpyrimidin (R = OMe)

Eine Suspension von 3 g (15 mmol) des Produktes aus Stufe a) in 35 ml POCl₃ wurde auf 88°C erhitzt. Dabei ging der Feststoff langsam in Lösung. Nach 5,5 h wurde das überschüssige Phosphoroxychlorid am Rotationsverdampfer abdestilliert und der sehr dickflüssige Rückstand auf Eis gegossen.
Dabei stieg die Temperatur auf 50°C an. Es wurden weitere 1,5 h gerührt. Nach Abkühlen auf 5°C wurde der Feststoff abfiltriert. Man erhielt 1,66 g (47%) Titelprodukt mit einem Gehalt von 97% (GC). Das Filtrat wurde drei Mal mit Essigester extrahiert, die gemeinsame organische Phase über Na₂SO₄ getrocknet und am Rotationsverdampfer eingeengt. Man erhielt weitere 0,65 g (18%) Titelprodukt mit einem Gehalt von 87% (nach GC).
- ¹H-NMR: (DMSO, 300 MHz) δ in ppm:: 3,7 (s, 3H);
7,62 (s, 2H);
8,8 (s, 1H);
9,1 (s, 1H).

### Beispiel 3:

### a) 5-(N-Trifluoracetyl)-2-amino-4,6-dihydroxypyrimidin (R = CF₃)

Zu einer 50°C warmen Lösung von 5,3 g (0,23 mol) Natrium in 600 ml Ethanol gab man 11,4 g (0,12 mol) Guanidinhydrochlorid und liess 10 min rühren. Zur weissen Suspension wurden innerhalb von 30 min 29,75 g (0,11 mol) N-Trifluoracetylaminomalonsäurediethylester zugegeben. Die dickflüssige Suspension wurde auf 80°C erhitzt und 4 h reagieren gelassen.
Anschliessend wurden weitere 4 g (40 mmol) Guanidinhydrochlorid zugegeben und eine weitere Stunde am Rückfluss erhitzt. Das Reaktionsgemisch wurde auf Raumtemperatur abgekühlt, filtriert und der Filterkuchen mit etwas Ethanol nachgewaschen. Der Feststoff wurde in 350 ml Wasser aufgenommen und mit konz. HCl wurde der pH auf ca. 5 gestellt. Die Suspension wurde im Eisbad gekühlt, filtriert und das Produkt am Vakuum bei 40°C getrocknet. Man erhielt 16,4 g (62%) violetten Feststoff.
- ¹H-NMR: (DMSO): 6,9 (br, s, 2H);
9,7 (s, 1H);
10,2-11,2 (br, 2H).

### b) 5-(N-Trifluoracetyl)-2-amino-4,6-dichlorpyrimidin (R = CF₃)

Eine Suspension von 15 g (63 mmol) des Produktes aus Stufe a) in 100 ml POCl₃ wurde während 2 h am Rückfluss erhitzt. Dabei ging der Feststoff allmählich in Lösung. Anschliessend wurde das Reaktionsgemisch auf Raumtemperatur abgekühlt, der Überschuss Phosphoroxychlorid abdestilliert und der dickflüssige Rückstand auf Eis gegossen. Dabei stieg die Temperatur bis auf 40°C an. Der pH wurde auf ca. 4 gestellt, und nach einer Stunde bei dieser Temperatur wurde das Produkt durch Extraktion mit 4 Portionen Essigester isoliert. Die gemeinsamen organischen Phasen wurden über Na₂SO₄ getrocknet und am Rotationsverdampfer eingeengt.
Man erhielt 5,97 g (35%) Titelprodukt.
- ¹H-NMR: (DMSO): 7,85 (s, 2H);
11,4 (s, 1H).

## Patentansprüche (Patentansprüche für folgende(n) Vertragsstaat(en): AT, BE, CH, LI, DE, DK, FR, GB, GR, IT, NL, PT, SE, IE)

1. N-5-geschützte 2,5-Diamino-4,6-dichlorpyrimidine der allgemeinen Formel 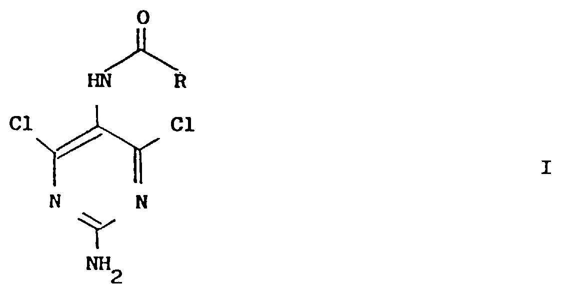 worin R eine C₁-C₄-Alkoxygruppe oder eine Trifluormethylgruppe bedeutet.

2. Verfahren zur Herstellung von N-5-geschützten 2,5-Diamino-4,6-dichlorpyrimidinen der allgemeinen Formel nach Patentanspruch 1, dadurch gekennzeichnet, dass ein N-geschützter Aminomalonsäureester der allgemeinen Formel 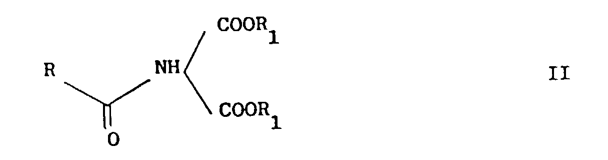 worin R₁ eine C₁-C₄-Niederalkylgruppe bedeutet und R die genannte Bedeutung hat, mit Guanidin in Gegenwart eines Alkalialkoholats zum N-5-geschützten 4,6-dihydroxy-2,5-diaminopyrimidin der allgemeinen Formel 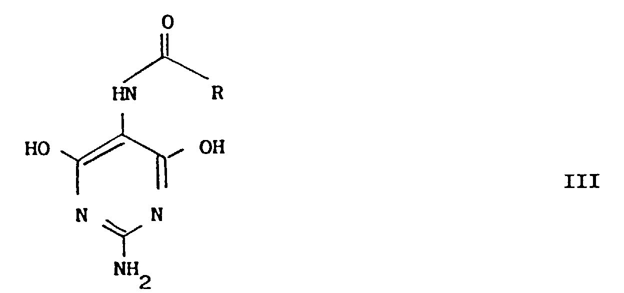 worin R die oben genannte Bedeutung hat, cyclisiert wird, und dieses Pyrimidin der Formel III anschliessend mit Phosphoroxychlorid zum Endprodukt umgesetzt wird.

3. Verfahren nach Patentanspruch 2, dadurch gekennzeichnet, dass die Cyclisierung des N-geschützten Aminomalonsäureesters in Gegenwart von Natriummethanolat in Methanol oder Natriumethanolat in Ethanol bei einer Temperatur zwischen Raumtemperatur und dem Siedepunkt des jeweiligen Alkohols erfolgt.

4. Verfahren nach Patentanspruch 2 oder 3, dadurch gekennzeichnet, dass die Chlorierung bei einer Temperatur zwischen 80°C und der Siedetemperatur des Phosphoroxychlorids erfolgt.

5. N-5-geschützte 2,5-Diamino-4,6-dihydroxypyrimidine der allgemeinen Formel 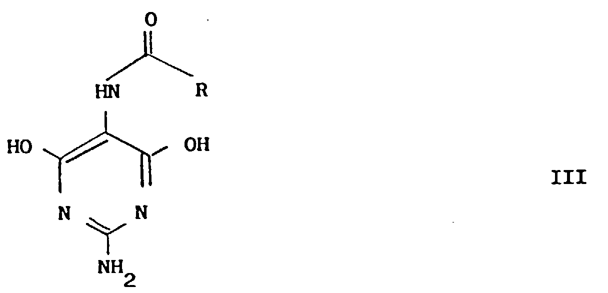 worin R eine C₁-C₄-Alkoxygruppe oder eine Trifluormethylgruppe bedeutet.

## Patentansprüche (Patentansprüche für folgende(n) Vertragsstaat(en): ES)

1. Verfahren zur Herstellung von N-5-geschützten 2,5-Diamino-4,6-dichlorpyrimidinen der allgemeinen Formel 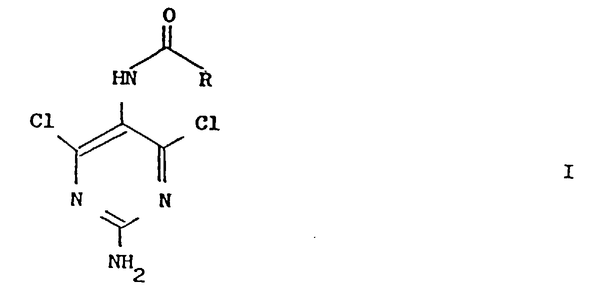 worin R eine C₁-C₄-Alkoxygruppe oder eine Trifluormethylgruppe bedeutet, dadurch gekennzeichnet, daß ein N-geschützter Aminomalonsäureester der allgemeinen Formel 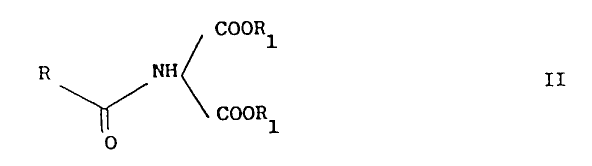 worin R₁ eine C₁-C₄-Niederalkylgruppe bedeutet und R die genannte Bedeutung hat, mit Guanidin in Gegenwart eines Alkalialkoholats zum N-5-geschützten 4,6-Dihydroxy-2,5-diaminopyrimidin der allgemeinen Formel 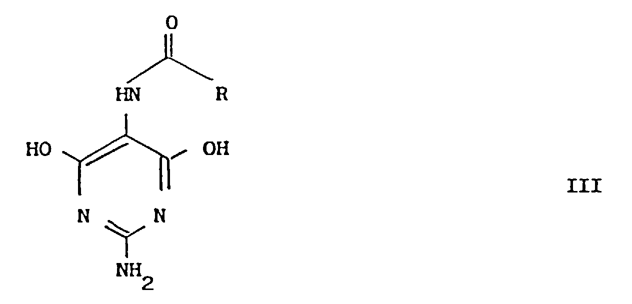 worin R die oben genannte Bedeutung hat, cyclisiert wird, und dieses Pyrimidin der Formel III anschließend mit Phosphoroxychlorid zum Endprodukt umgesetzt wird.

2. Verfahren nach Anspruch 1,dadurch gekenzeichnet, daß die Cyclisierung des N-geschützten Aminomalonsäureesters in Gegenwart von Natriummethanolat in Methanol oder Natriummethanolat in Ethanol bei einer Temperatur zwischen Raumtemperatur und dem Siedepunkt des jeweiligen Alkohols erfolgt.

3. Verfahren nach Patentanspruch 1 oder 2, dadurch gekennzeichnet, daß die Chlorierung bei einer Temperatur zwischen 80°C und der Siedetemperatur des Phosphoroxychlorids erfolgt.

## Claims (Claims for the following Contracting State(s): AT, BE, CH, LI, DE, DK, FR, GB, GR, IT, NL, PT, SE, IE)

1. N-5-protected 2,5-diamino-4,6-dichloropyrimidines of the general formula 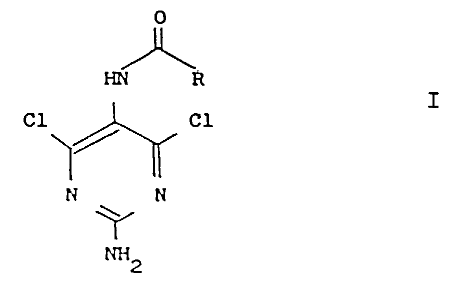 in which R represents a C₁-C₄-alkoxy group or a trifluoromethyl group.

2. Process for preparing N-5-protected 2,5-diamino-4,6-dichloropyrimidines of the general formula given in Claim 1, characterised in that an N-protected aminomalonate of the general formula 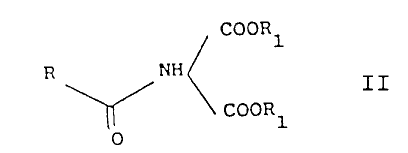 in which R₁ represents a C₁-C₄-lower-alkyl group and R has the meaning given above, is cyclised with guanidine in the presence of an alkali metal alcoholate to give the N-5-protected 4,6-dihydroxy-2,5-diaminopyrimidine of the general formula 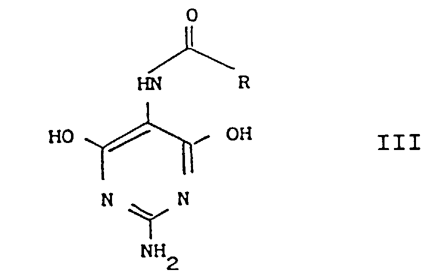 in which R has the meaning given above, and this pyrimidine of the formula III is then reacted with phosphorus oxychloride to give the end product.

3. Process according to Claim 2, characterised in that cyclisation of the N-protected aminomalonate is performed in the presence of sodium methanolate in methanol or sodium ethanolate in ethanol at a temperature between room temperature and the boiling point of the particular alcohol.

4. Process according to Claim 2 or 3, characterised in that chlorination is performed at a temperature between 80°C and the boiling point of phosphorus oxychloride.

5. N-5-protected 2,5-diamino-4,6-dihydroxypyrimidines of the general formula 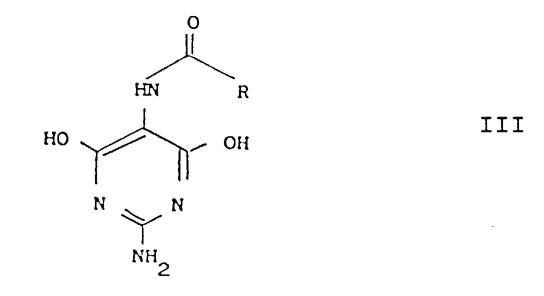 in which R represents a C₁-C₄-alkoxy group or a trifluoromethyl group.

## Claims (Claims for the following Contracting State(s): ES)

1. Process for preparing N-5-protected 2,5-diamino-4,6-dichloropyrimidines of the general formula 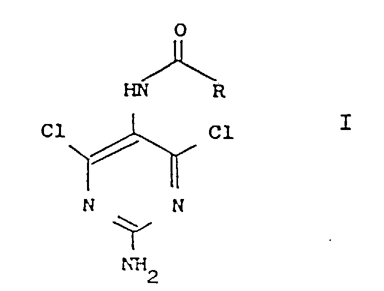 in which R represents a C₁-C₄-alkoxy group or a trifluoromethyl group, characterised in that an N-protected aminomalonate of the general formula 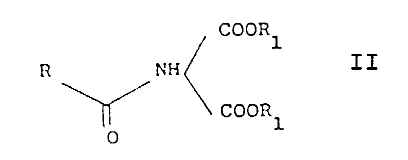 in which R₁ represents a C₁-C₄-lower-alkyl group and R has the meaning given above, is cyclised with guanidine in the presence of an alkali metal alcoholate to give the N-5-protected 4,6-dihydroxy-2,5-diaminopyrimidine of the general formula 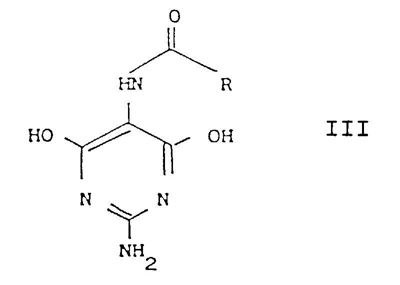 in which R has the meaning given above, and this pyrimidine of the formula III is then reacted with phosphorus oxychloride to give the end product.

2. Process according to Claim 1, characterised in that cyclisation of the N-protected aminomalonate is performed in the presence of sodium methanolate in methanol or sodium ethanolate in ethanol at a temperature between room temperature and the boiling point of the particular alcohol.

3. Process according to Claim 1 or 2, characterised in that chlorination is performed at a temperature between 80°C and the boiling point of phosphorus oxychloride.

## Revendications (Revendications pour l'(les) Etat(s) contractant(s) suivant(s): AT, BE, CH, LI, DE, DK, FR, GB, GR, IT, NL, PT, SE, IE)

1. 2,5-diamino-4,6-dichloropyrimidines N-5 protégées de la formule générale 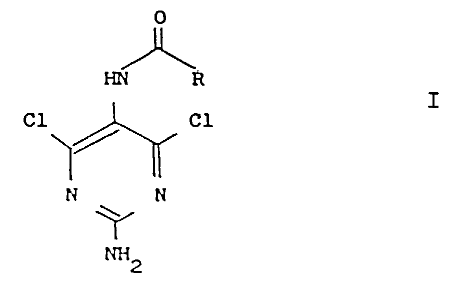 dans laquelle R signifie un groupe alcoxy C₁-C₄ ou un groupe trifluorométhyle.

2. Procédé pour la préparation de 2,5-diamino-4,6-dichloropyrimidines N-5 protégés de la formule générale selon la revendication 1, caractérisé en ce qu'un ester de l'acide aminomalonique N-protégé de la formule générale 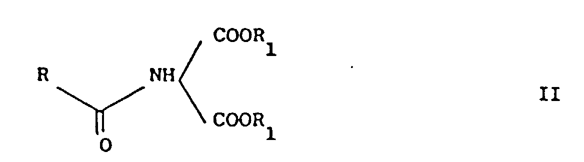 dans laquelle R₁ signifie un groupe alkyle inférieur C₁-C₄ et R a la signification indiquée ci-dessus, est cyclisé avec la guanidine en présence d'un alcoolat alcalin en 4,6-dihydroxy-2,5-diaminopyrimidine N-5 protégée de la formule générale 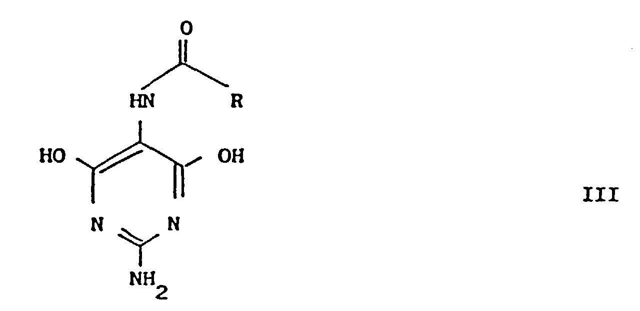 dans laquelle R a la signification précitée, et cette pyrimidine de la formule III est consécutivement transformée en produit final avec l'oxychlorure de phosphore.

3. Procédé selon la revendication 2, caractérisé en ce que la cyclisation de l'ester de l'acide aminomalonique protégé N s'effectue en présence d'éthanolat de sodium dans le méthanol ou de méthanolat de sodium dans l'éthanol à une température entre la température ambiante et le point d'ébullition de l'alcool respectif.

4. Procédé selon la revendication 2 ou 3, caractérisé en ce que la chloruration s'effectue à une température entre 80°C et la température d'ébullition de l'oxychlorure de phosphore.

5. 2,5-diamino-4,6-dihydroxypyrimidines N-5 protégées de la formule générale 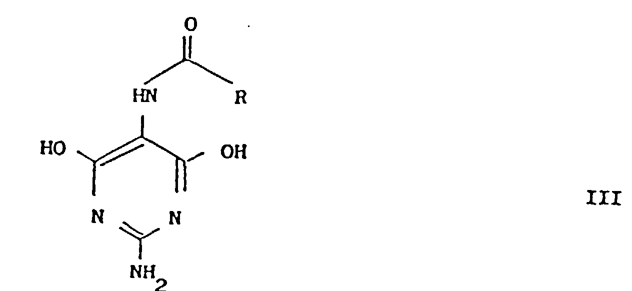 dans laquelle R signifie un groupe alcoxy C₁-C₄ ou un groupe trifluorométhyle.

## Revendications (Revendications pour l'(les) Etat(s) contractant(s) suivant(s): ES)

1. Procédé pour la préparation de 2,5-diamino-4,6-dichloropyrimidines protégées N-5 de la formule générale 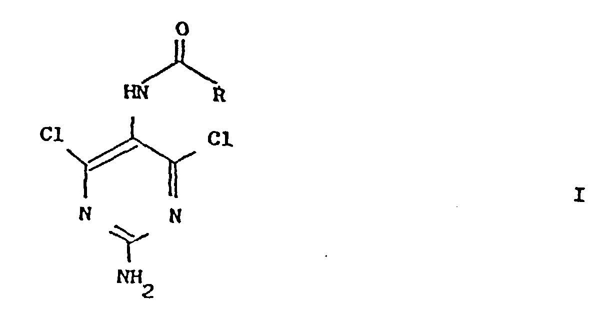 dans laquelle R signifie un groupe alcoxy C₁-C₄ ou un groupe trifluorométhyle, caractérisé en ce qu'un ester de l'acide aminomalonique protégé N de la formule générale 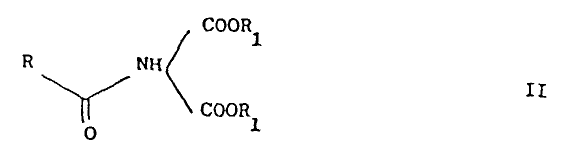 dans laquelle R₁ signifie un groupe alkyle inférieur C₁-C₄ et R a la signification ci-dessus, est cyclisé avec la guanidine en présence d'un alcoolat alcalin en 4,6-dihydroxy-2,5-diaminopyrimidine N-5 protégée de la formule générale 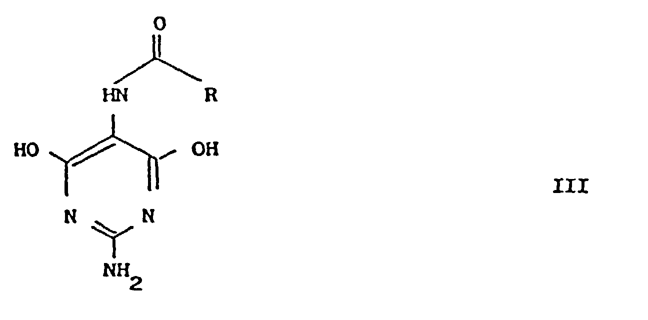 dans laquelle R a la signification ci-dessus, et cette pyrimidine de la formule III est consécutivement transformée en produit final avec l'oxychlorure de phosphore.

2. Procédé selon la revendication 1, caractérisé en ce que la cyclisation de l'ester de l'acide aminomalonique N protégé s'effectue en présence d'éthanolat de sodium dans le méthanol ou de méthanolat de sodium dans l'éthanol à une température entre la température ambiante et le point d'ébullition de l'alcool respectif.

3. Procédé selon la revendication 1 ou 2, caractérisé en ce que la chloruration s'effectue à une température entre 80°C et la température d'ébullition de l'oxychlorure de phosphore.
